# EUROPEAN PATENT SPECIFICATION

(11) **EP 4 013 304 B1**
(45) Date of publication and mention of the grant of the patent: **19.02.2025**
(21) Application number: 20754263.0
(22) Date of filing: 14.08.2020
(51) Int. Cl.: A61B 5/145, A61B 5/1455, A61B 5/00

(54) **ACTIVE MINIATURIZED SENSING SYSTEM AND METHOD**
AKTIVES MINIATURISIERTES ERFASSUNGSSYSTEM UND VERFAHREN
SYSTÈME ET MÉTHODE DE DÉTECTION

(30) Priority: 16.08.2019 US 201962887767 P; 16.08.2019 EP 19192138; 06.09.2019 EP 19195939; 19.12.2019 EP 19218195; 21.02.2020 EP 20158808
(43) Date of publication of application: 22.06.2022
(73) Proprietor: Glucomat GmbH, 93309 Kelheim (DE)
(72) Inventor: REICHL, Mathias, 93326 Abensberg (DE)
(74) Representative: Weickmann & Weickmann PartmbB
(86) International application number: PCT/EP2020/072885
(87) International publication number: WO 2021/032629

(56) References cited:
- US-A- 5 313 941
- US-A- 5 900 632
- US-A1- 2011 009 720
- US-A1- 2016 334 332
- ZHENG P ET AL: "NONINVASIVE GLUCOSE DETERMINATION BY OSCILLATING THERMAL GRADIENT SPECTROMETRY", DIABETIS TECHNOLOGY & THERAPEUTICS, THOMSON REUTERS, MARY ANN LIEBERT, LARCHMONT, NY; US, vol. 2, no. 1, 1 March 2000 (2000-03-01), pages 17 - 25, XP009010296, ISSN: 1520-9156, DOI: 10.1089/152091500316692

## Description

The present invention relates to a non-invasive active sensing system for determining glucose in a bodily fluid of subject. Further, the present invention relates to a non-invasive method for determining glucose in a bodily fluid of a subject.

### Background

In the year 2016, about 415 million people suffered from diabetes. For 2040, an increase to more than 640 million people can be expected. Since people with diabetes are at risk for complications such as blindness, kidney diseases, heart diseases and stroke, there is a need to control the disease by closely monitoring blood glucose level.

Presently, determination of blood glucose is mainly based on invasive system and methods, wherein either a blood sample is taken and subsequently subjected to an *in vitro* test or a sensor is implanted for determining the glucose level *in vivo.* These invasive systems and methods are disadvantageous in that they are painful or inconvenient.

Thus, there is a need for developing a system and methods which allow a reliable non-invasive determination of glucose and or physiological parameters.

Pertinent prior art to understand the contribution of the invention is disclosed in the following documents: US 5313941A (BRAIG ET AL, 1994-05-24), US 2011/009720 A1 (KUNJAN ET AL, 2011-01-13), US 2016/334332 A1 (MAGNUSSEN ET AL, 2016-11-17), US 5900632 A (STERLING ET AL, 1999-05-04) and the scientific article from ZHENG ET AL with title "NONINVASIVE GLUCOSE DETERMINATION BY OSCILLATING THERMAL GRADIENT SPECTROMETRY" (2000-03-01, DOI:10.1089/152091500316692). These documents disclose glucometers based on infrared signal sensing.

### Summary of the invention

The present invention is defined by appended claims 1-13.

According to the present disclosure, a simple, rapid and reliable determination of glucose is feasible using non-invasive systems and methods. These systems and methods involve irradiation of a body part of a subject, particular a human subject, with visual (VIS)/near-infrared (NIR) radiation in the range of about 400 nm to about 1500 nm or about 500 nm to about 1500 nm and detecting emitted IR radiation from the irradiated body part of said subject in the range of about 5 µm to about 12 µm. Surprisingly, the present inventor has found that irradiating a body part, e.g. a fingertip, an earlobe, a wrist or a forearm with short-wavelength radiation and detecting emitted long-wavelength radiation from the irradiated body part allows determination for physiological parameters such as glucose in a bodily fluid such as blood.

Irradiation of a body part with VIS/NIR radiation according to the present invention causes energy absorption within an area of the irradiated body part. Energy absorption in this irradiated area, i.e. the absorption area, results in a local increase of tissue temperature within the irradiated body part, particularly within the absorption area, which again causes an increased emission of IR radiation from the irradiated body part, particularly from the absorption area, including an increased emission of IR radiation in the range of about 5 µm to about 12 µm. Thus, detection of emitted IR radiation from the irradiated body part is facilitated and substantially improved.

A first aspect of the invention relates to a non-invasive system for determining glucose in a bodily fluid of a subject comprising:
(a) a radiation source (9, 9a, 9b) adapted to emit visible (VIS)/near-infrared (NIR) radiation in the range of 400 nm to 1500 nm or 500 nm to 1500 nm into a body part (1) of said subject,
   wherein the body part (1) is particularly selected from a fingertip, an ear lobe, a wrist, a forearm, and upper arm,
   and wherein the irradiated body part absorbs the emitted electromagnetic energy resulting in a local increase of tissue temperature and in an increased emission of IR radiation in the wavelength range of 5 µm to 12 µm,
(b) a sensing unit adapted to detect emitted IR radiation from the previously irradiated body part of said subject in the range of 5 µm to 12 µm,
   wherein said sensing unit is adapted to (i) detect IR radiation having at least one first wavelength or first wavelength range where the intensity of the detected IR-radiation is dependent from the concentration of glucose in the bodily fluid of said subject, and to (ii) detect IR radiation having at least one second wavelength or second wavelength range where the intensity of the detected IR radiation is substantially independent from the concentration of glucose in the bodily fluid of said subject,
   wherein the at least one first wavelength or first wavelength range and the at least one second wavelength or second wavelength range are within the wavelength range of 5 µm to 12 µm and
(c) an analyzing unit configured to perform the qualitative and/or quantitative determination of the glucose based on the IR radiation detected in the sensing unit (b) in the at least one first wavelength or first wavelength range and the at least one second wavelength or second wavelength range, wherein the sensing unit (b) comprises at least two first sensors (4),

wherein each of the at least two first sensors (4) is adapted to detect IR radiation having at least one first wavelength or first wavelength range where the intensity of the detected IR radiation is dependent from the concentration of glucose in the bodily fluid of said subject, and
wherein at least one first sensor (4) is adapted to detect IR radiation having a wavelength of about 9.2 µm and at least one other first sensor (4) is adapted to detect IR radiation having a wavelength range between 9.2 µm and 9.6 µm which comprises the first wavelength of about 9.2 µm and further comprises a wavelength of about 9.4 µm and about 9.6 µm,
   and
wherein the sensing unit comprises at least one second sensor (4), which is adapted to detect IR radiation having at least one second wavelength or second wavelength range where the intensity of the detected IR radiation is substantially independent from the concentration of glucose in the bodily fluid of said subject.

A still further aspect of the invention relates to a method for non-invasively determining glucose in a bodily fluid of a subject comprising the steps:
(a) irradiating a body part (1) of said subject with visible (VIS)/near-infrared (NIR) radiation in the wavelength range of 400 nm to 1500 nm, or 500 nm to 1500 nm, wherein the irradiated body part (1) absorbs the emitted electromagnetic energy resulting in a local increase of tissue temperature and in an increased emission of IR radiation in the wavelength range of 5 µm to 12 µm,
(b) detecting emitted IR radiation from the previously irradiated body part of said subject in the wavelength range of 5 µm to 12 µm,
   comprising separately (i) detecting IR radiation having at least two wavelengths or wavelength ranges where the intensity of the detected IR radiation is dependent from the concentration of glucose in the bodily fluid of said subject, comprising detecting IR radiation having a wavelength of about 9.2 µm and detecting IR radiation having a wavelength range between 9.2 µm and 9.6 µm which comprises the wavelength of about 9.2 µm and further comprises a wavelength of about 9.4 µm and about 9.6 µm,
      and
   (ii) detecting IR radiation having at least one wavelength or wavelength range where the intensity of the detected IR radiation is substantially independent from the concentration of glucose in the bodily fluid of said subject, and
(c) analyzing the detected IR radiation for the qualitative and/or quantitative determination of glucose,
particularly wherein the concentration of glucose is quantitatively determined, and/or wherein the alteration rate of the amount of glucose is determined, particularly non-quantitatively determined.

### Detailed description

The present invention involves determination of glucose by detecting IR radiation from previously irradiated body parts of a subject, particularly a human subject in the wavelength range of about 5 µm to about 12 µm, particularly in the range of about 8 µm to about 10 µm.

In the invention, the system is adapted for the non-invasive determination of glucose in blood. In this embodiment, IR radiation is detected at a glucose-specific wavelength or wavelength range where glucose has a characteristic absorption band and where the intensity of the detected IR radiation is dependent from the concentration of glucose in the blood. More particularly, the glucose-specific wavelength or wavelength range is selected from a combination of a wavelength of about 9.2 µm and a wavelength range comprising all three of the wavelengths of about 9.2 µm, about 9.4 µm and about 9.6 µm. Additionally, IR radiation is detected at a reference wavelength or wavelength range where glucose has no characteristic absorption band and particularly an absorption minimum and where the intensity of the detected IR radiation is substantially independent from the concentration of glucose in blood. More particularly, the reference wavelength or wavelength range is selected from a wavelength or wavelength range between about 8.7 µm to about 9.0 µm, a wavelength or a wavelength range between about 9.7 µm to about 10.2 µm or any combination thereof.

As outlined above, the invention is based on the irradiation of body tissue with electromagnetic radiation in the wavelength range between about 500 nm to about 1500 nm (VIS/NIR radiation) and detection of electromagnetic radiation emitted from the irradiated body part in the wavelength range between about 5 µm to about 15 µm (IR radiation). Irradiation of the body part with VIS/NIR radiation results in an enhanced self-emission of IR radiation from said body part due to local energy absorption, which causes a local increase in temperature. Thus, self-emission of IR radiation from the irradiated body part is increased by previous irradiation of said body part with VIS/NIR radiation. Consequently, irradiation of the body part with an external source of IR radiation in the wavelength range between about 5 µm to about 15 µm is not required. Thus, in certain embodiments, the system of the invention does not include an external IR radiation source, particularly in certain embodiments the system of the invention does not include an external IR radiation source adapted to irradiate the body part from which the detected IR radiation is emitted.

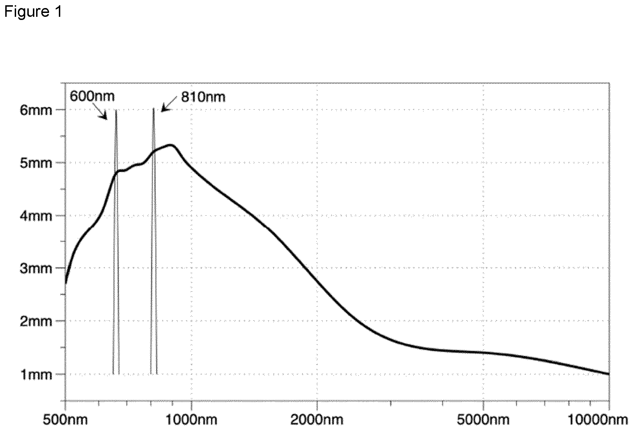

**Figure** 1 shows the penetration depth of electromagnetic radiation into body tissue [mm] depending from the wavelength [nm]. It can be seen that the penetration depth is dependent from the wavelength. In the visual (VIS)/near-infrared (NIR) wavelength range between about 400 nm to about 1500 nm, particularly in the range of about 500 nm to about 1500 nm or in the range of about 400 nm to about 1200 nm, more particularly in the range of about 550 nm to about 1200 nm, there is a penetration depth of about 1 mm or more, particularly about 3 mm or more. Thus, a body part irradiated with radiation will absorb electromagnetic energy resulting in a local increase of tissue temperature. This again results in an increased emission of longer-wavelength IR radiation, e.g. IR radiation in the wavelength range of about 5 µm to about 12 µm, where certain organic compounds present in bodily fluids, i.e. physiological parameters, show absorption bands. This allows a quantitative or qualitative determination of glucose according to the above aspects of the present invention.

In certain embodiments, the VIS/NIR radiation emitted into the body part is in the range of about 550 nm to about 1000 nm, particularly in the range of about 800 nm to about 820 nm, e.g. about 810 nm, and/or in the range of about 590 nm to about 660 nm, e.g. at about 600 nm, and/or in the range of about 920 nm to about 980 nm, e.g. at about 940 nm. In certain embodiments, the VIS/NIR radiation emitted into the body is in the range of about 450 nm to about 800 nm.

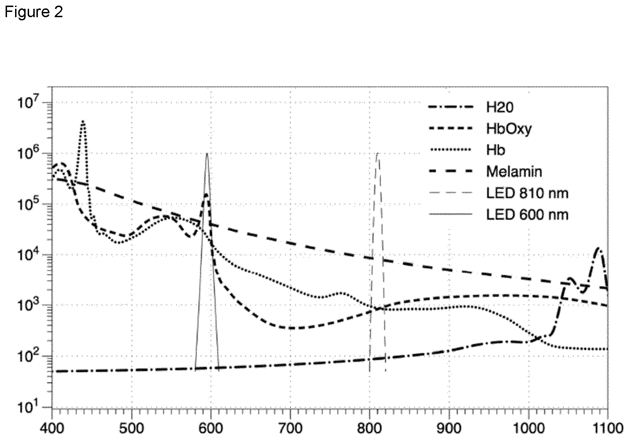

**Figure 2** shows relative absorption coefficients of certain compounds present in the human body depending from the wavelength in the range between 400 nm and 1100 nm. The wavelengths of about 600 nm and about 810 nm, at which radiation may be emitted into the body part, are specifically indicated. In the wavelength range of about 500 nm to about 1050 nm, there is a relatively low absorption of water (H₂O). Further, the major blood constituents hemoglobin (Hb) and oxyhemoglobin (Hboxy) show similar absorption coefficients. The skin pigment melamine shows an absorption coefficient which decreases with increasing wavelength.

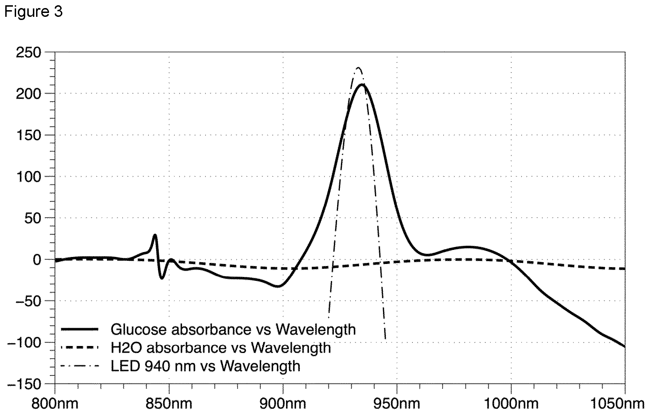

In an embodiment of the invention, the radiation source (a) is adapted for emitting VIS/NIR radiation in the range of about 920 nm to about 960 nm, e.g. about 940 nm into a body part. This irradiation wavelength may be used alone or in combination with at least one further irradiation wavelength. As shown in **Figure 3****,** glucose has an absorption band at a wavelength of 940 nm. Thus, irradiation at a wavelength of about 940 nm leads to a selective excitation of glucose molecules and may result in a stronger absorption of glucose molecules in the IR wavelength range, particularly in the wavelength range of about 5 µm to about 12 µm.

According to an embodiment of the invention, the radiation source (a) is adapted for emitting VIS/NIR radiation in the range of about 920 nm to about 980 nm, e.g. about 940 nm into a body part of said subject, and the sensing unit (b) is further adapted for detecting VIS/NIR radiation having a wavelength of about 940 nm, where the intensity of the detected VIS/NIR radiation is dependent from the concentration of glucose. The measurement signal in the VIS/NIR wavelength range may be combined with the measurement signals in the IR range as described above, e.g. by means of a comparator.

In a still further embodiment, VIS/NIR irradiation occurs at a combination of at least 2 different wavelengths, particularly at a combination of a first wavelength of about 800 nm to about 820 nm, e.g. about 810 nm, and a second wavelength of about 920 nm to about 980 nm, e.g. about 940 nm.

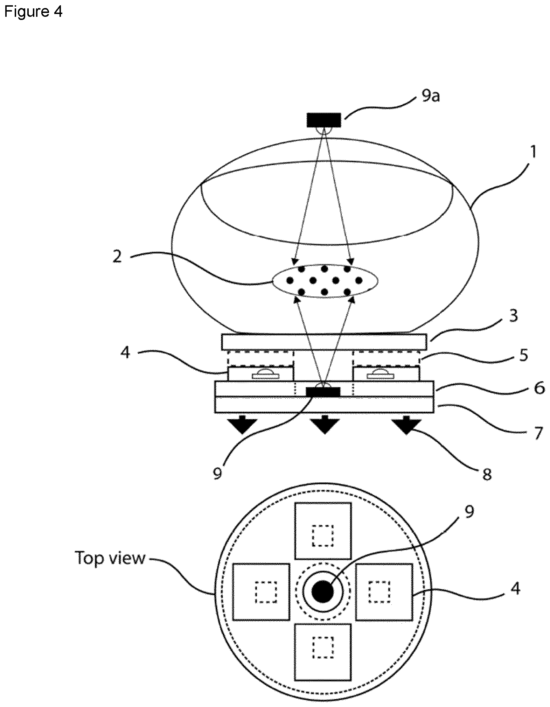

**Figure 4** shows an embodiment of a system of the present invention. A body part (1), e.g. a fingertip, is placed into contact with the system, which is adapted to irradiate an absorption area (2) within the body part (1).

The system comprises a cover (3) which is at least partially made of a material, which is optically transparent. For example, the cover is at least partially made of CaF₂ and/or BaF₂ or of a plastic material which is transparent in the IR wavelength range of about 5 µm to about 12 µm or a sub-range thereof, e.g. of about 8 µm to about 12 µm, and which is optionally transparent in the VIS/NIR wavelength range of about 400 nm to about 1500 nm or a sub-range thereof. Suitable IR-transparent plastic materials are e.g. the PolyIR plastic materials commercially available from Fresnel Technologies, Fort Worth, Texas, USA. In certain embodiments, the cover may have a thickness of about 0.2 mm to about 2 mm, particularly of about 0.5 mm to about 1.5 mm, more particularly about 1 mm.

The system further comprises at least one sensor (4) which may be provided with a filter element (5) and optionally a lens element (not shown), which e.g. may be arranged between a sensor (4) and a filter element (5). The sensor (4) may be mounted on a circuit board (6). Further, the system comprises at least one radiation source (9, 9a). For example, the system may comprise a radiation source (9) located on the same side as the sensor (4) and/or a radiation source (9a) located on an opposite side of the body part (1) with regard to the sensor (4). If desired, a further sensor (4) may be provided without filter element (5) for monitoring the exact skin temperature of the subject.

The system contains one or more sensors (4). In the embodiment of Figure 4, the system comprises four different sensors (4). The sensor may be an optical detector, particularly an optical photovoltaic detector, e.g. an InAsSb-based detector, which may be used in combination with a lock-in amplifier, if desired. A photovoltaic detector, e.g. an InAsSb-based detector has a rise time of only few nanoseconds and is particularly useful in a set-up wherein the body part is irradiated intermittently. In other embodiments, the sensor may be heat detector, e.g. a thermopile or a bolometer. Suitable sensors include a photovoltaic detector (e.g. Hamamatsu P13894), a thermopile (e.g. Heimann HCS C21 F8-14) or other types of IR sensors (e.g. Sensirion STS21 or Melexis MLX90632). If desired, a sensor (4) may be provided with a filter element (5) capable of selectively transmitting radiation of a desired wavelength or wavelength range. The filter element may have narrow bandwidth, e.g. of about 50 to 100 nm, or a broader bandwidth, e.g. of about 400 nm or more. A filter may be made from germanium or other filter materials, which are optically transmissive for the respective wavelengths. Further, a sensor may be provided with a lens element, e.g. a micro-lens capable of focusing the light falling onto the sensor.

In certain embodiments, the sensor surface may be coated with a noble metal such as Au or Ag, particularly Au, in order to increase its sensitivity. Such a coating which may be shaped as a Bundt baking-pan is described by Awad (Nature Scientific Reports 9:12197 (2019)), the content of which is herein incorporated by reference.

In certain embodiments, the sensor is a miniaturized sensor having an area of about 1 mm² to about 10,000 mm², e.g. of about 10 mm² to about 1,000 mm². In certain embodiments, the sensor may be even more miniaturized, e.g. an ASIC (application-specific integrated circuit).

In the sensing unit of the invention, at least two sensors are analyte-specific sensors, i.e. sensors which are adapted for detecting IR radiation having at least one wavelength or wavelength range where the intensity of the detected IR radiation is dependent from the concentration of glucose in the bodily fluid of said subject, and at least on sensor may be a reference sensor, i.e. a sensor which is adapted for detecting IR radiation having at least one wavelength or wavelength range where the intensity of the detected IR radiation is substantially independent from the concentration of glucose in the bodily fluid of said subject.

The sensing unit (b) is adapted for detecting self-emitted IR radiation from the previously irradiated body part, i.e. IR radiation generated by the body heat of the subject without irradiation by an external IR source.In the invention, the sensing unit (b) is adapted for detecting emitted IR radiation from an absorption area within the previously irradiated body part wherein the absorption area has a locally increased temperature and exhibits an increased emission of IR radiation in the wavelength range of about 5 µm to about 12 µm.

In certain embodiments, at least one further sensor may be present, e.g. a sensor which (i) is adapted for detecting unspecific IR radiation, (ii) is adapted for detecting unspecific VIS/NIR radiation, (iii) is adapted for detecting VIS/NIR radiation having a wavelength, where the intensity of the detected VIS/NIR radiation is dependent from the concentration of the physiological parameter in the bodily fluid of said subject, and/or (iv) a temperature sensor for measuring the temperature of the body part.

In certain embodiments, at least one further analyte-specific sensor may be present, i.e. a sensor which is adapted for detecting VIS/NIR radiation having at least one wavelength or wavelength range where the intensity of the detected VIS/NIR radiation is dependent from the concentration of the physiological parameter in the bodily fluid of said subject. For example, at least one further sensor adapted for detecting VIR/NIR radiation having a wavelength of about 940 nm may be present.

Further, the device may comprise a circuit board (7) on which the light source (9) is mounted and an active and/or passive heat sink (8).

The VIS/NIR radiation source (9, 9a, 9b) may be adapted for emitting collimated radiation, e.g. a laser-based light source, and/or adapted for emitting non-collimated radiation, e.g. an LED-based light source. For example, the light source may be selected from an LED, a laser diode, a VCSEL (vertical-cavity surface-emitting laser) or a laser. In certain embodiments, a broadband VIS/NIR radiation emitter is used which may be adapted for emitting VIS/NIR radiation in the range of about 650 nm to about 950 nm, particularly in the range of about 750 nm to about 850 nm and more particularly in the range of about 780 nm to about 820 nm. Suitable VIS/NIR emitters are e.g. the OSLON products from Osram such as OSLON SFH 4763.

In the claimed invention, the radiation source is adapted for emitting VIS/NIR radiation in the range of about 400 nm to about 1500 nm, particularly in the range of about 500 nm to about 1500 nm. The VIS/NIR radiation may be emitted continuously or intermittently throughout a predetermined time interval.

In the claimed invention, the radiation source is adapted to cause a local increase in the temperature of the irradiated body part, e.g. a fingertip, and particularly a local increase in the temperature of the absorption area within the irradiated body part. The local increase in temperature may be in the range of between about 1°C to about 15°C, particularly about 2°C to about 10°C, and more particularly in the range of about 3°C to about 5°C. The locally increased temperature of the irradiated body part, e.g. a fingertip, may be in temperature range up to about 45°C, up to about 40°C or up to about 37°C, for example in the temperature range between about 30°C to about 35°C or about 30°C to about 32°C. This local temperature increase results in an enhanced self-emission of IR radiation from the irradiated body part and particularly from the absorption area within the irradiated body part.

In a certain embodiment, the radiation source may be adapted for emitting radiation continuously at a power of about 10 mW to about 1 W, particularly for about 20 mW to about 500 mW, more particularly of about 50 mW to about 250 mW, and even more particularly of about 100 mW to about 200 mW, e.g. about 150 mW, for a time interval of about 0.1 to about 20 s, particularly of about 0.2 s to about 5 s, and more particularly of about 0.5 s to about 2 s, e.g. about 1 s.

In a further embodiment, the radiation source may be adapted for emitting radiation intermittently at a power of about 10 mW to about 5 W, particularly of about 20 mW to about 1 W, and more particularly of about 50 mW to about 500 mW for a time interval of about 0.1 s to about 20 s, particularly of about 0.2 s to about 5 s, and more particularly of about 0.5 s to about 2 s. The radiation may be emitted intermittently with a pulse frequency of about 1 Hz to about 1 MHz.

In a further embodiment, the radiation source may be adapted to emit VIS/NIR radiation at a plurality of different wavelengths, e.g. at 2, 3, 4, 5, 6, 7, 8 or even more different wavelengths. For example, the radiation source may be multi LED chip. The use of a multi-wavelength radiation source allows adjusting a predetermined penetration depth of electromagnetic radiation into the tissue of the irradiated body part depending on specific characteristics of the body part, e.g. pigmentation, skin thickness, presence or absence of horny skin. As shown in Figure 1, supra, the penetration depth into body tissue varies with the wavelength and the use of VIS/NIR radiation with different wavelengths or with combinations of different wavelengths can be adapted for each subject and/or each body part individually, if desired.

In certain embodiments, the radiation source (a) is a multi-wavelength radiation source is adapted to emit VIS/NIR radiation at several different wavelengths or wavelength ranges, for example, between about 400 nm to about 1200 nm, more particularly between about 450 nm and about 900 nm, e.g. at least 2, 3 4, 6 or 8 wavelengths which may be selected from wavelengths at about 470 nm, about 520 nm, about 590 nm, about 650 nm, about 750 nm and about 810 nm.

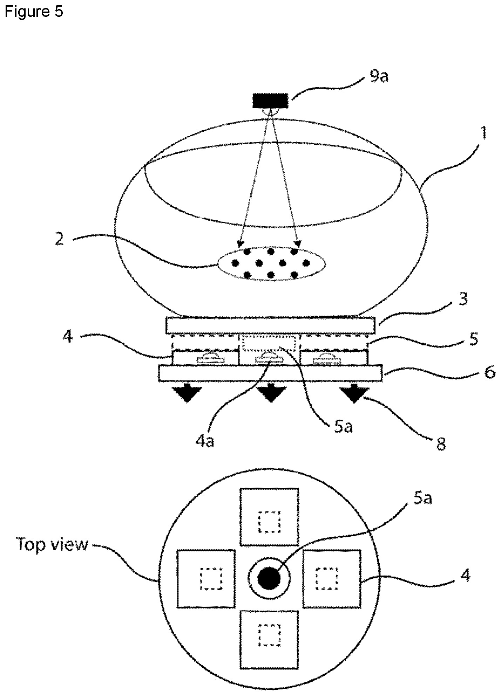

A further embodiment of the system of the invention is shown in **Figure 5****.** Here, a single radiation source (9a) is provided on a side of the body part (1) which is opposite to a sensing unit comprising at least one sensor (4) provided with a filter (5) and a further sensor (4a) provided with a filter (5a). In certain embodiments, sensor (4a) is an optical sensor, e.g. a photodiode. It is adapted for a reference measurement of transmission radiation from radiation source (9a), e.g. for measuring radiation at a wavelength of about 600 nm and/or about 810 nm and/or about 940 nm. For this purpose, filter element (5a) may be a bandpass filter at about 600 nm and/or 810 nm and/or 940 nm.

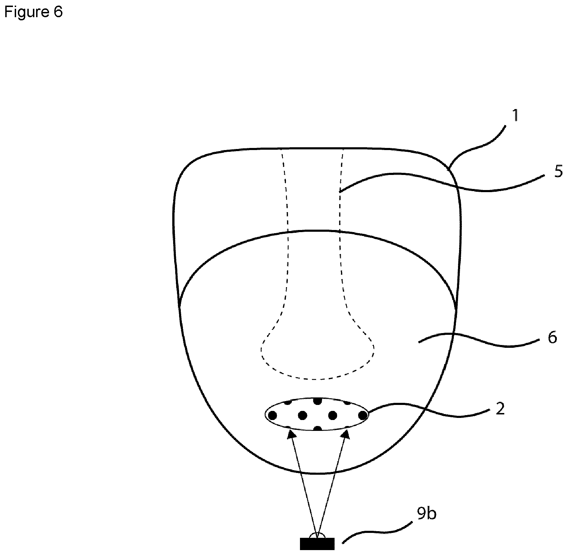

Still a further embodiment of the invention is shown in **Figure 6****.** Here, a radiation source (9b) is provided on a side of the body part (1), e.g. a fingertip, wherein direct access to an absorption area (2) within the body part (1) is provided through the skin of the body part without the radiation passing through a cover structure of the device and/or without passing through a horny structure on the body surface, e.g. a finger nail and/or horny skin. Thereby, interference, e.g. interference from the cover structure or from keratinic horny skin or nail material and optionally nail varnish can be reduced or eliminated. According to this embodiment, a single radiation source (9b) or a plurality of radiation sources (9b), e.g. 2, 3, 4, 6 or 8 radiation sources may be provided at a position around the circumference of the body part (1), e.g. a fingertip. If a plurality of radiation sources is present, they are preferably adapted to emit radiation into a single absorption area (2) within the body part, which may be about 3 mm to about 5 mm below the body surface.

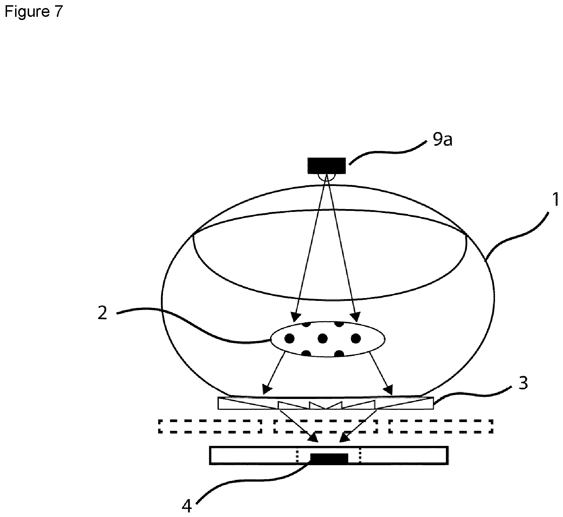

Still a further embodiment of the invention is shown in **Figure 7****.** In this embodiment, a cover (3) is provided which is adapted for focusing IR radiation emitted from the body part to the at least one sensor (4) of the sensing unit. Thereby, the radiation intensity on the sensor and thus the sensitivity and/or accuracy of the measurement may be increased. The cover (3) is made of a material, e.g. plastic, metal, metal oxide or composite material, which is substantially transparent for IR radiation in the wavelength range to be detected on the sensor, particularly for IR radiation in the wavelength range of about 5 µm to about 12 µm or a sub-range thereof, e.g. of about 8 µm to about 12 µm. Suitable materials are e.g. the PolyIR plastic materials, c.f. supra. In this embodiment, the cover (3) may comprise an IR Fresnel lens, i.e. a lens of large aperture and short focal length capable of efficiently focusing IR radiation passing therethrough, or an array comprising a plurality e.g. up to 10 or more IR Fresnel lenses. In certain embodiments, the array may comprise IR Fresnel micro-lenses, e.g. up to 100 or 1000 micro-lenses, which may have diameters in the range of about 50 nm to about 500 µm. In certain embodiments, the IR Fresnel lens may have a back focal length of about 3 mm to about 10 mm, e.g. about 5 mm and may be manufactured from an IR-transparent plastic. For example, a suitable IR Fresnel lens, which is optically transparent in the wavelength range of 8-14 µm is commercially available from Edmund Optics (product family no. 2042).

Further, Figure 7 shows a radiation source (9a) provided on the opposite side of the body part with regard to the position of the sensing unit, which comprises a sensor (4). It should be noted, however, that one or more radiation sources may also be arranged in a circumferential arrangement around the body part (1), e.g. as shown in Figure 6. It should further be noted, that in this embodiment a plurality of different sensors may be present, e.g. as shown in Figures 4 and 5.

As shown in Figures 4 and 5, the system of the invention comprises a plurality of different glucose-specific sensors wherein a first sensor is adapted for detecting radiation at a first wavelength of about 9.2 µm and at least another first sensor is adapted for detecting IR radiation at a wavelength of about 9.2 µm and additionally at a wavelength of about 9.4 µm and a wavelength of about 9.6 µm.

Further, the sensing unit may comprise a plurality of reference sensors adapted for detecting reference radiation at different wavelengths or wavelength ranges. For example, when determining glucose, a reference sensor may be adapted for detecting radiation having a wavelength range between about 8.6 µm and about 9.0 µm. Another reference sensor is adapted for detecting radiation at a wavelength or wavelength range between about 9.8 µm and about 10.2 µm.

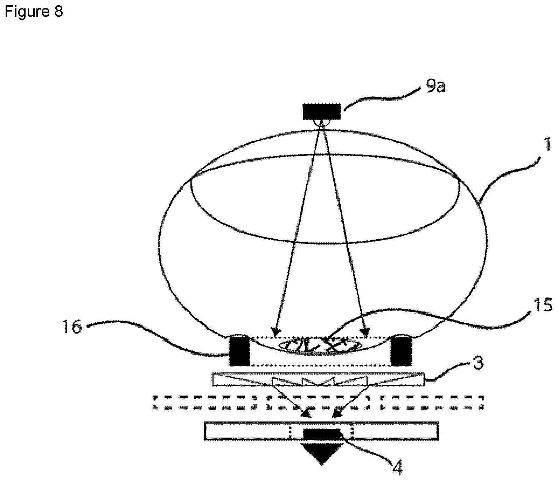

Still a further embodiment of the invention is shown in **Figure 8****.** In this embodiment, a support (16) for the body part (1), e.g. a fingertip, is provided wherein said support (16) comprises an opening adapted to receive a portion (15) of the body part (1). For example, the support may comprise an annular structure with an opening, e.g. a substantially circular opening, in its center. The system is adapted for pressing the body part (1) onto the opening in the support (16) such that a portion (15) of the body part (1), e.g. a portion of the fingertip, is forced into the opening. Thus, the tissue including the blood vessels within portion (15) is compressed resulting in an enhanced amount of capillary blood within portion (15). Thereby, the signal intensity and thus the sensitivity and/or accuracy of the measurement may be increased.

Further, the system of Figure 8 includes a cover (3) which may be formed as an IR Fresnel lens as described above in the context of Figure 7. It should be noted, however, that other covers are also suitable. Furthermore, a radiation source (9a) is shown which is provided on the opposite side of the body part with regard to the position of the sensing unit, which comprises a sensor (4). It should be noted, however, that one or more radiation sources may also be arranged in a circumferential arrangement around the body part (1), e.g. as shown in Figure 6. It should further be noted, that in this embodiment a plurality of different sensors may be present, e.g. as shown in Figures 4 and 5.

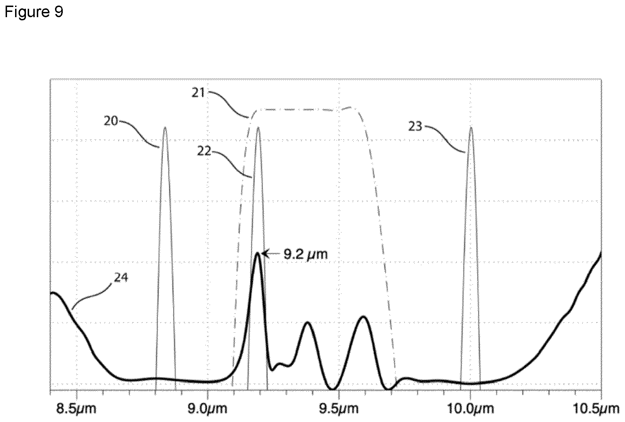

In **Figure 9****,** measurement at a plurality of analyte-specific wavelengths/wavelength ranges and reference wavelengths/wavelength ranges is shown.

The absorption signal of glucose (24) has three different peaks at about 9.2 µm, at about 9.4 µm and about 9.6 µm. A first glucose-specific sensor may be adapted for measuring only the peak at 9.2 µm. Such a sensor would be adapted with a filter element capable of transmitting radiation only in a narrow range (22). Thus, the sensor is capable of selectively detecting radiation within this narrow range. A further glucose-specific sensor may be adapted for measuring radiation at a broader range between about 9.1 µm and about 9.7 µm, thereby encompassing the peaks at about 9.2 µm, 9.4 µm and 9.6 µm. This sensor may be adapted with a filter element capable of transmitting radiation in a broader range (21).

Two reference sensors may be provided, wherein said reference sensors are provided with filter elements capable of transmission of radiation with a wavelength in the range of about 8.6 µm and about 9.0 µm, particularly of about 8.8 µm - 8.9 µm (20) and/or radiation with a wavelength in the range of about 9.8 µm and about 10.2 µm, particularly of about 9.9 µm - 10.1 µm (23), respectively.

Parallel and separate measurements at a wavelength of about 9.2 µm on the one hand and at a wavelength range including the peak at 9.2 µm, but also at least one of the other peaks, particularly the peak at about 9.6 µm have a further advantage, since they allow determination whether the subject's blood contains ethanol. Since ethanol and other alcohols have an absorption band at a wavelength of about 9.6 µm, but not at a wavelength of about 9.2 µm, the ratio between the peak at 9.2 µm and 9.6 µm may be used to determine and optionally correct a disturbance caused by blood alcohol.

In an alternative embodiment, a first glucose-specific sensor may be adapted for measuring only the peak at 9.6 µm. Such a sensor would be provided with a filter element capable of transmitting radiation only in a narrow range. A further glucose-specific sensor may be adapted for measuring radiation at a broader range between about 9.4 µm and about 9.6 µm, thereby encompassing the peaks at about 9.4 µm and about 9.6 µm and not encompassing the peak at 9.2 µm. This sensor may be provided with a filter element capable of transmitting radiation in a broader range.

In a further alternative embodiment, a reference sensor may be provided, which is provided with a filter element capable of transmission of radiation with a wavelength in the range of about 7.8 µm and about 8.2 µm, particularly of about 7.9 µm - 8.1 µm, optionally in combination with at least one further reference sensor, which is provided with a filter element capable of transmission of radiation with a wavelength in the range of about 8.8 µm - 9.2 µm and/or radiation with a wavelength of about 9.8 µm - 10.2 µm, respectively

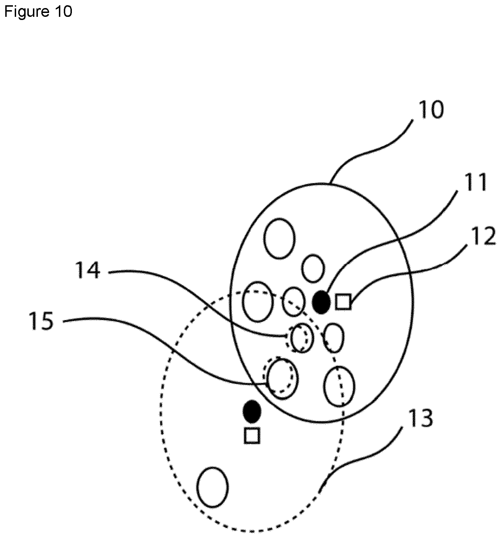

In a still further embodiment of the invention, the system may comprise a sensor, which is adapted for a time-dependent detection of IR radiation having different wavelengths or wavelength ranges. In this embodiment, the system may be provided with a sensor comprising a plurality of filters adapted for transmitting IR radiation having different wavelengths or wavelength ranges wherein said filters may be placed on a sensor during different stages of a measurement cycle thereby allowing detection of different wavelengths or wavelength ranges within a measurement cycle. Such an embodiment is shown in **Figure 10****.** Here, a system is provided comprising a filter wheel (10) capable of rotating around an axis (11) and a shutter wheel (13) capable of rotating around an axis. The filter wheel and the shutter wheel are provided with illumination holes (15) through which light from the radiation source (not shown) may pass into the body part of the subject (not shown). Reflected light from the irradiated body part may pass through different holes (14) of the filter wheel (10) which may be provided with analyte-specific filter elements and/or reference filter elements as described above. The filter wheel's (10) and the shutter wheel's (13) position may be monitored with a magnet (12) in combination with a magnetic sensor. In operation, they may be rotated with predetermined frequencies, thereby allowing time-dependently passing of radiation from the radiation source into the body part and time-dependently passing of radiation emitted from the body part at predetermined time intervals through the different holes (14) of the filter wheel (10) to a sensor (not shown).

In an alternative embodiment (not shown), a sensor which is adapted for a time-dependent detection of IR radiation having different wavelengths or wavelength ranges, may be a Fabry-Perot interferometer, e.g. a MEMS spectrometer for the desired IR wavelength range (cf. Tuohinieni et al., J. Micromech. Microeng. 22 (2012), 115004; Tuohinieni et al., J. Micromech. Microeng. 23 (2013), 075011).

In certain embodiments, the system comprises a single sensor, which is adapted for a time-dependent detection of IR radiation having different wavelengths or wavelength ranges. This sensor may be provided with different filters, e.g. with a filter wheel, or be a Fabry-Perot interferometer as described above.

The system of the present invention further comprises an analyzing unit (c) for the qualitative and/or quantitative determination of a physiological parameter based on the IR radiation detected in the sensing unit (b). The analyzing unit may comprise, for example, an A/D converter and a microcontroller. The analysis of the measured signal may be based on the intensity and/or the decay time.

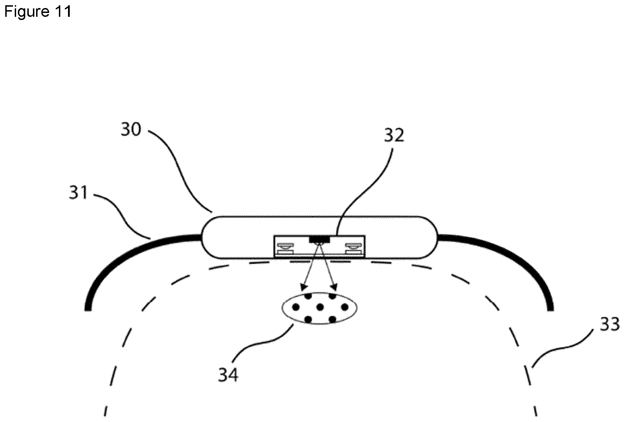

A still further embodiment of the invention is shown in **Figure 11****.** The system of this embodiment is adapted for being permanently fixed to the subject's body. This system is particularly adapted for carrying out a plurality of measurements in predetermined time intervals. The system comprises a housing (30) and a strap (31) for fixing the housing around the body (33), e.g. a wrist or forearm. Further, the system comprises a radiation source for emitting VIS/NIR light into an absorption area (34) of the body part (33) and sensors for detecting IR radiation emitted from the irradiated body part.

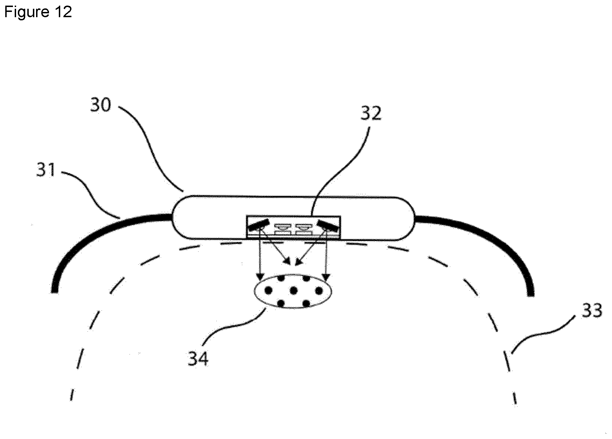

A still further embodiment of the invention is shown in **Figure 12****.** The system of this embodiment is adapted for being permanently fixed to the subject's body and particularly adapted for carrying out a plurality of measurements in predetermined time intervals. The system comprises a housing (30) and a strap (31) for fixing the housing around the body (33), e.g. a wrist or forearm. Further, the system comprises a plurality of radiation sources, e.g. 2 radiation sources, for emitting VIS/NIR light into an absorption area (34) of the body part (33) and sensors for detecting IR radiation emitted from the irradiated body part. The light emitted from these sources may fall at angle, e.g. at an angle of about 30° to about 75° onto the surface of the body part (33).

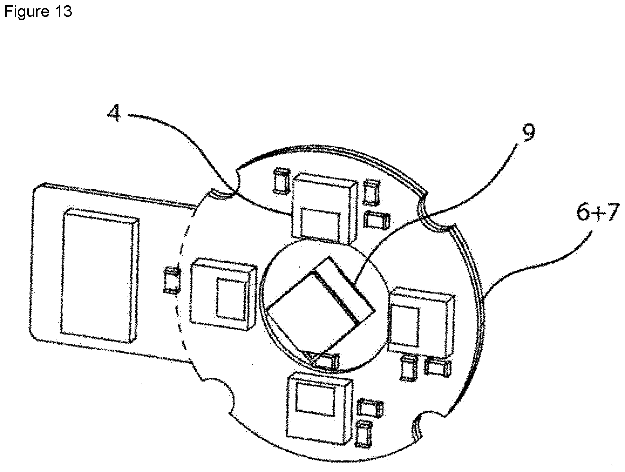

In **Figure 13****,** a schematic view of the system of Figure 4 is shown.

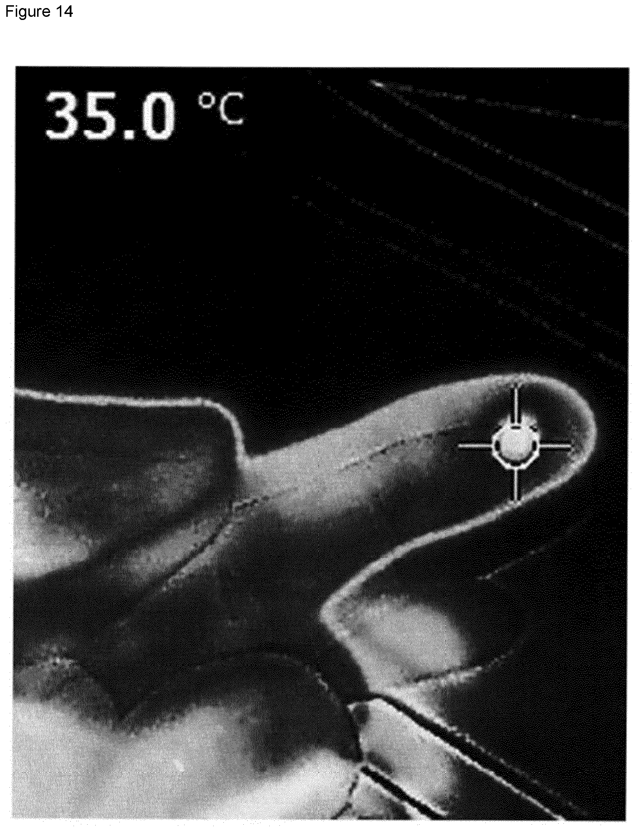

**Figure 14** shows a heat map of a fingertip after irradiation with light of 810 nm for a time period of 2 s.

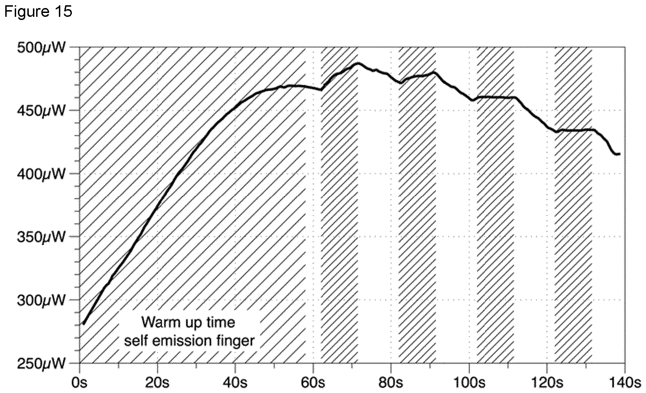

**Figure 15** is diagram showing the time-dependent thermal power output in addition to the self-emission of a fingertip during intermittent irradiation with light of 810 nm with a power of 2 mW and a frequency of 0.1 Hz.

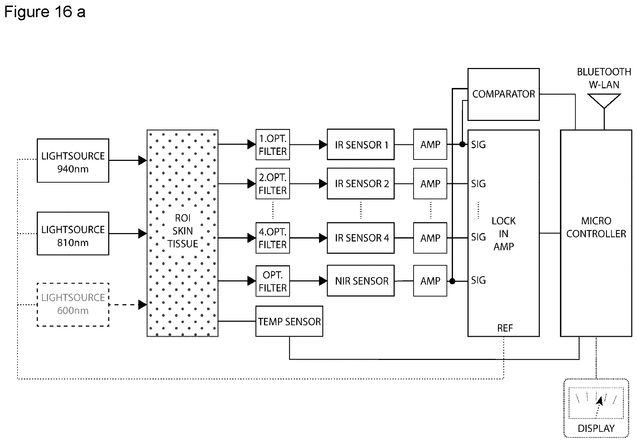

**Figure 16a** shows a block diagram of an embodiment of the sensing unit the present invention. A Region of Interest (ROI), i.e. the skin tissue of a subject, particularly a human subject, is irradiated with a first light source emitting VIS/NIR radiation having a wavelength of 940 nm, a second light source emitting VIS/NIR radiation having a wavelength of about 810 nm and optionally a third light source emitting VIS/NIR radiation having a wavelength of about 600 nm. Radiation transmitted through the Region of Interest or reflected from the Region of Interest is analyzed by a sensing unit. Further, the device comprises a temperature sensor.

The sensing unit comprises a plurality of sensors, for example analyte-specific IR sensors (1) and (2) and reference sensors, e.g. IR sensor (4). For the determination of glucose, an IR sensor (1) may be provided with a first optical filter, which is transmissive for a wavelength of about 9.2 µm and an IR sensor (2) may be provided with a second optical filter, which is transmissive for a wavelength range between about 9.2 µm and about 9.6 µm. A reference sensor (4) may be provided with a fourth optical filter which is transmissive for a wavelength or wavelength range between about 8.6 µm and about 9.0 µm and/or a wavelength or wavelength range between about 9.8 µm and about 10.2 µm. Further, the sensing unit comprises an NIR sensor for detecting VIS/NIR radiation having a wavelength of about 940 µm where glucose has a strong absorption band. The NIR sensor is provided with a suitable optical filter, which is transmissive for this wavelength. Further, the sensing unit may comprise a temperature sensor for measuring the temperature of the skin tissue in the Region of Interest. The respective sensors may be coupled to amplifiers (AMP) for first signal amplification. Signals from individual sensors may be referenced with signals from other sensors by means of a comparator, thereby improving the measurement accuracy and/or signal quality. For example, the measurement signal from the NIR sensor at 940 nm may be referenced with the measurement signal from analyte-specific IR sensor (1). Alternatively or additionally, the measurement signal from the NIR sensor at 940 nm may be referenced with the measurement signals from analyte-specific IR sensor (1) and/or analyte-specific IR sensor (2) and/or reference IR sensor (4). The measured and optionally referenced signals are further amplified by a lock-in amplifier unit and transmitted to a microcontroller unit. A feedback control from the lock-in amplifier to the light sources may be provided. From the microcontroller unit, the signal and/or the result of internal algorithms may be transmitted to a display unit and/or another device, e.g. by a direct connection or via Bluetooth and/or WLAN.

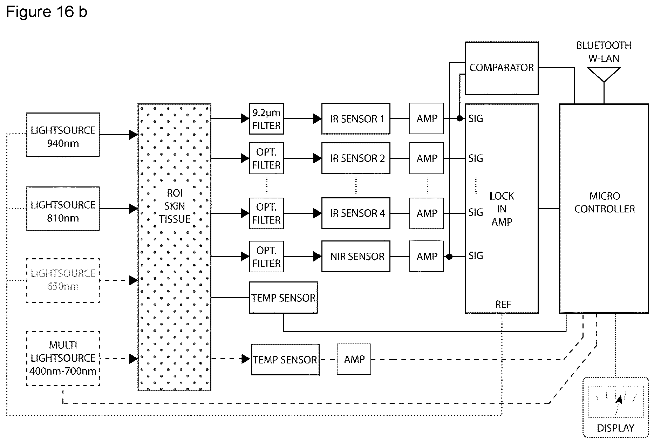

**Figure 16b** shows a block diagram of a further embodiment of the sensing unit of the present invention, which is similar to the sensing unit shown in Fig. 16a. Here, additionally or alternatively, a multi-wavelength light source, e.g. a multi-wavelength LED comprising a plurality of individual diodes is provided. The multi-wavelength light source may e.g. have a wavelength range from 400 nm to about 700 nm is provided and may be operated by the microcontroller unit. Further, a temperature sensor coupled to an amplifier (AMP) is present. This temperature sensor may also be operated by the microcontroller unit.

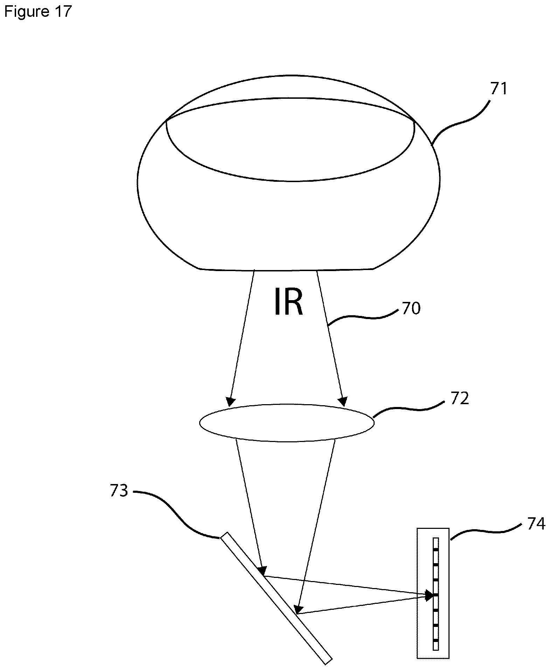

In a still further embodiment of the invention, the system may comprise a spectral or line sensor or spectral or line sensor array, typically a bolometer or thermopile array, which is adapted for detecting an IR spectrum within the wavelength range of interest, e.g. including the range of about 7 µm to about 12 µm, particularly including the range of about 8 µm to about 10 µm. An IR spectrum may be generated by passing the IR radiation from the irradiated body part through a spectral splitting or diffracting device and then to the sensor or sensor array. Such an embodiment is shown in **Figure 17****.** Emitted IR radiation (70) from the irradiated body part (71), e.g. a fingertip, is optionally focused by a focusing element (72) adapted for focusing IR radiation, e.g. a lens or concave mirror element, and then passed to an spectral splitting or diffracting element (73), e.g. a prism or an transmissive or reflective optical grating, where the IR radiation is split according to its wavelength. From there the diffracted radiation is passed to a spectral sensor or line sensor or sensor array (74), typically a bolometer or thermopile array, where an IR spectrum in the wavelength range of interest, e.g. between 8 µm and about 20 µm including analyte-specific wavelengths or wavelength ranges and reference wavelengths or wavelength ranges, e.g. as described above, is detected. The amount of the physiological parameter of interest, e.g. glucose may be determined by spectral analysis according to the relative intensities of predetermined analyte-specific and reference wavelengths.

The system and the method of the invention allow qualitative and/or quantitative determination of the physiological parameter to be measured, the qualitative and/or quantitative determination of glucose in blood.

In certain embodiments, the concentration of the physiological parameter, the concentration of glucose in blood, is quantitatively determined. In certain embodiments, the alteration rate of the measured amount of the physiological parameter, glucose, is determined. These embodiments may involve a non-quantitative measurement, e.g. a relative measurement of the alteration of the analyte amount per time unit, i.e. the increase of the analyte amount or the decrease of the analyte amount per time unit. In case the alteration of the analyte amount into a single direction, i.e. increase or decrease, exceeds a predetermined level and/or time period, the system will provide an alert. This embodiment is particularly useful for systems as shown in Figure 11 and Figure 12, which may be permanently fixed at the body of the subject, e.g. around the wrist, forearm or upper arm. This embodiment may be adapted for steady glucose level monitoring.

In certain embodiments, the system of the invention is adapted for performing both non-quantitative measurements and quantitative measurements. For example, the system may be adapted for performing non-quantitative measurements, e.g. qualitatively measuring the alteration, e.g. the increase or decrease, of the analyte amount over time during standard operation. Non-quantitative measurements may e.g. be performed as continuous and/or intermittent monitoring measurements, as required. In case the alteration of the analyte amount exceeds a predetermined level and/or time period, the system is adapted to switch to a quantitative measurement in order to provide more detailed information. In these embodiments, a system adapted to be permanently fixed to the body, e.g. to an arm wrist, or to an ankle, may be used. Specific embodiments of such systems are shown in Figure 11 and Figure 12.

**In** certain embodiments, the system is adapted to perform non-quantitative measurements, e.g. continuous and/or intermittent monitoring measurements, and quantitative measurements on several different body parts. For example, the system may be adapted to perform non-quantitative measurements on a first body part, e.g. a body part to which the system may be permanently fixed, such as an arm wrist or an ankle, and to perform quantitative measurements on a second body part, e.g. a body part where capillary vessels are more accessible, such as an earlobe or a fingertip. For performing a measurement on the second body part, the system is removed from the first body part and brought into contact, particularly into direct contact with the second body part. After performing the measurement on the second body part, the system may be removed therefrom and brought again into contact with the first body part, e.g. by fixing the system to the first body part. **In** specific embodiments, the first body part is an arm wrist and/or the second body part is a fingertip.

A still further aspect of the invention is a non-invasive system for determining glucose in blood, which allows identification and optional correction of disturbances caused by blood alcohol comprising a sensing unit for detecting emitted **IR** radiation from a body part of said subject in the range of about 5 µm to about 12 µm,
wherein said sensing unit is adapted for (i) detecting **IR** radiation at a wavelength of about 9.2 µm and separately therefrom for detecting **IR** radiation at a wavelength range encompassing the wavelength of about 9.2 µm, about 9.4 µm and about 9.6 µm, and an analyzing unit for the separate determination of glucose from the above sensing units.

Still a further aspect of the invention is a method for non-invasively determining glucose in blood of a subject using this system.

Preferred features of these aspects are as previously indicated in the above specification.

Still a further aspect of the present disclosure is the use of an InAsSb sensor, optionally in combination with a lock-in amplifier for the measure of **IR** radiation emitted from a body part.

Preferred features of this aspect are as previously indicated in the above specification.

Still a further aspect of the present disclosure is a system and method for the non-quantitative measurement of glucose involving a plurality of measurements during a predetermined time interval and determining an alteration of the measurement signal indicating an alteration of the amount of analyte and providing an alter if the alteration of the glucose amount to one direction, i.e. increase or decrease, exceeds a certain level in a predetermined time period. This system and method may be adapted for steady glucose level monitoring.

Preferred features of this aspect are as previously indicated in the above specification.

The scope of the invention is defined by the appended claims.

## Claims

1. A non-invasive system for determining glucose in a bodily fluid of a subject comprising:
(a) a radiation source (9, 9a, 9b) adapted to emit visible (VIS)/near-infrared (NIR) radiation in the range of 400 nm to 1500 nm or 500 nm to 1500 nm into a body part (1) of said subject,
wherein the body part (1) is particularly selected from a fingertip, an ear lobe, a wrist, a forearm, and upper arm,
and wherein the irradiated body part absorbs the emitted electromagnetic energy resulting in a local increase of tissue temperature and in an increased emission of IR radiation in the wavelength range of 5 µm to 12 µm,
(b) a sensing unit adapted to detect emitted IR radiation from the previously irradiated body part of said subject in the range of 5 µm to 12 µm,
wherein said sensing unit is adapted to (i) detect IR radiation having at least one first wavelength or first wavelength range where the intensity of the detected IR-radiation is dependent from the concentration of glucose in the bodily fluid of said subject, and to (ii) detect IR radiation having at least one second wavelength or second wavelength range where the intensity of the detected IR radiation is substantially independent from the concentration of glucose in the bodily fluid of said subject,
wherein the at least one first wavelength or first wavelength range and the at least one second wavelength or second wavelength range are within the wavelength range of 5 µm to 12 µm and
(c) an analyzing unit configured to perform the qualitative and/or quantitative determination of glucose based on the IR radiation detected in the sensing unit (b) in the at least one first wavelength or first wavelength range and the at least one second wavelength or second wavelength range,
wherein the sensing unit (b) comprises at least two first sensors (4),
wherein each of the at least two sensors (4) is adapted to detect IR radiation having at least one first wavelength or first wavelength range where the intensity of the detected IR radiation is dependent from the concentration of glucose in the bodily fluid of said subject, and
wherein at least one first sensor (4) is adapted to detect IR radiation having a wavelength of about 9.2 µm and at least one other first sensor (4) is adapted to detect IR radiation having a wavelength range between 9.2 µm and 9.6 µm which comprises the first wavelength of about 9.2 µm and further comprises a wavelength of about 9.4 µm and about 9.6 µm,
and
wherein the sensing unit comprises at least one second sensor (4), which is adapted to detect IR radiation having at least one second wavelength or second wavelength range where the intensity of the detected IR radiation is substantially independent from the concentration of glucose in the bodily fluid of said subject.

2. The system of claim 1, which does not comprise an external radiation source adapted to emit IR radiation in the wavelength range of 5 µm to 12 µm.

3. The system of claim 1 or 2, wherein the bodily fluid is blood.

4. The system of any one of the preceding claims, wherein the radiation source (a) is adapted to emit VIS/NIR radiation in the range of 550 nm to 1200 nm, particularly in the range of 800 nm to 820 nm, e.g., at about 810 nm, and/or in the range of 590 nm to 610 nm, e.g., at about 600 nm, and/or in the range of 920 nm to 980 nm, e.g., at about 940 nm.

5. The system of any one of the preceding claims, wherein the radiation source (a) is a LED, a laser diode, a vcsel (vertical-cavity surface-emitting laser) or a laser, and/or wherein the radiation source (a) is a multi-wavelength radiation source.

6. The system of claim any one of the preceding claims, further comprising at least one third sensor,
wherein the at least one third sensor (i) is adapted to detect unspecific IR radiation, (ii) is adapted to detect unspecific VIS/NIR radiation, (iii) is adapted to detect VIS/NIR radiation having a wavelength, where the intensity of the detected VIS/NIR radiation is dependent from the concentration of the physiological parameter in the bodily fluid of said subject, and/or (iv) is a temperature sensor adapted to measure the temperature of the body part.

7. The system of any one of the preceding claims comprising at least two different second sensors, which are adapted to detect IR radiation having at least two different wavelengths or wavelength ranges,
wherein the system is particularly adapted to determine glucose in blood, wherein a second sensor is adapted to detect IR radiation having a wavelength or wavelength range between 8.6 µm and 9.0 µm and another second sensor is adapted to detect IR radiation having a wavelength or wavelength range between 9.8 µm and 10.2 µm.

8. The system of any of the preceding claims, wherein the sensing unit (b) comprises at least one sensor adapted to time-dependently and separately detect IR radiation having different wavelengths or wavelength ranges,
wherein in at least one first time interval the sensor is adapted to detect IR radiation having at least one wavelength or wavelength range where the intensity of the detected IR radiation is dependent from the concentration of glucose in the bodily fluid of said subject, and
wherein in at least one second time interval the sensor is adapted to detect IR radiation having at least one wavelength or wavelength range where the intensity of the detected IR radiation is substantially independent from the concentration of glucose in the bodily fluid of said subject.

9. The system of any one of the preceding claims,
wherein the sensing unit (b) comprises at least one sensor which is an optical detector, particularly an optical photovoltaics detector, more particularly an InAsSb-based detector.

10. The system of any one of the preceding claims further comprising a cover, wherein said cover is at least partially made of a material which is optically transparent for IR/VIS radiation emitted by the radiation source (a) and for IR radiation detected by the sensing unit (b),
wherein the cover is at least partially made of CaF₂ and/or BaF₂ and and/or of a plastic material which is transparent for IR radiation and optionally transparent for VIS/NIR radiation,
wherein the optically transparent material of the cover has a thickness of about 0.2 mm to about 2 mm, particularly of about 0.5 mm to about 1.5 mm, more particularly about 1 mm, and/or further comprising a cover which focuses IR radiation from the body part to the sensing unit (b), particularly to the at least one sensor of the sensing unit (b), particularly wherein the cover comprises an IR Fresnel lens or an array comprising a plurality of IR Fresnel lenses.

11. A method for non-invasively determining glucose in a bodily fluid of a subject comprising the steps:
(a) irradiating a body part (1) of said subject with visible (VIS)/near-infrared (NIR) radiation in the wavelength range of 400 nm to 1500 nm, or 500 nm to 1500 nm, wherein the irradiated body part (1) absorbs the emitted electromagnetic energy resulting in a local increase of tissue temperature and in an increased emission of IR radiation in the wavelength range of 5 µm to 12 µm,
(b) detecting emitted IR radiation from the previously irradiated body part of said subject in the wavelength range of 5 µm to 12 µm,
comprising separately (i) detecting IR radiation having at least two wavelengths or wavelength ranges where the intensity of the detected IR radiation is dependent from the concentration of glucose in the bodily fluid of said subject, comprising detecting IR radiation having a wavelength of about 9.2 µm and detecting IR radiation having a wavelength range between 9.2 µm and 9.6 µm which comprises the wavelength of about 9.2 µm and further comprises a wavelength of about 9.4 µm and about 9.6 µm,
and
(ii) detecting IR radiation having at least one wavelength or wavelength range where the intensity of the detected IR radiation is substantially independent from the concentration of glucose in the bodily fluid of said subject, and
(c) analyzing the detected IR radiation for the qualitative and/or quantitative determination of glucose,
particularly wherein the concentration of glucose is quantitatively determined, and/or wherein the alteration rate of the amount of glucose is determined, particularly non-quantitatively determined.

12. The method of claim 11, wherein the body part is not irradiated with an external source of IR radiation in the wavelength range of 5 µm to 12 µm.

13. The method of claim 11 or 12, wherein the bodily fluid is blood.

## Patentansprüche

1. Nicht-invasives System zur Bestimmung von Glukose in einer Körperflüssigkeit eines Subjekts, umfassend:
(a) eine Strahlungsquelle (9, 9a, 9b), die dazu ausgelegt ist, sichtbare (VIS)/nahe Infrarot (NIR)-Strahlung im Bereich von 400 nm bis 1500 nm oder 500 nm bis 1500 nm in ein Körperteil (1) des Subjekts zu emittieren,
wobei das Körperteil (1) insbesondere aus einer Fingerspitze, einem Ohrläppchen, einem Handgelenk, einem Unterarm und einem Oberarm ausgewählt ist,
und wobei das bestrahlte Körperteil die emittierte elektromagnetische Energie absorbiert, was zu einem lokalen Anstieg der Gewebetemperatur und zu einer erhöhten Emission von IR-Strahlung im Wellenlängenbereich von 5 µm bis 12 µm führt,
(b) eine Sensoreinheit, die so ausgelegt ist, dass sie die von dem zuvor bestrahlten Körperteil des Subjekts emittierte IR-Strahlung im Bereich von 5 µm bis 12 µm erfasst, wobei die Sensoreinheit so ausgelegt ist, dass sie (i) IR-Strahlung mit mindestens einer ersten Wellenlänge oder einem ersten Wellenlängenbereich erfasst, wo die Intensität der erfassten IR-Strahlung von der Glukosekonzentration in der Körperflüssigkeit des Subjekts abhängt, und dass sie (ii) IR-Strahlung mit mindestens einer zweiten Wellenlänge oder einem zweiten Wellenlängenbereich erfasst, wo die Intensität der erfassten IR-Strahlung im Wesentlichen unabhängig von der Glukosekonzentration in der Körperflüssigkeit des Subjekts ist,
wobei die mindestens eine erste Wellenlänge oder der erste Wellenlängenbereich und die mindestens eine zweite Wellenlänge oder der zweite Wellenlängenbereich innerhalb des Wellenlängenbereichs von 5 µm bis 12 µm liegen und
(c) eine Analyseeinheit, die so ausgelegt ist, dass sie die qualitative und/oder quantitative Bestimmung von Glukose auf der Grundlage der in der Sensoreinheit (b) erfassten IR-Strahlung bei der mindestens einen ersten Wellenlänge oder in dem ersten Wellenlängenbereich und bei der mindestens einen zweiten Wellenlänge oder in dem zweiten Wellenlängenbereich durchführt,
wobei die Sensoreinheit (b) mindestens zwei erste Sensoren (4) umfasst,
wobei jeder der mindestens zwei Sensoren (4) dazu ausgelegt ist, IR-Strahlung mit mindestens einer ersten Wellenlänge oder einem ersten Wellenlängenbereich zu erfassen, wo die Intensität der erfassten IR-Strahlung von der Glukosekonzentration in der Körperflüssigkeit des Subjekts abhängig ist, und
wobei mindestens ein erster Sensor (4) dazu ausgelegt ist, IR-Strahlung mit einer Wellenlänge von etwa 9,2 µm zu erfassen, und mindestens ein anderer erster Sensor (4) dazu ausgelegt ist, IR-Strahlung mit einem Wellenlängenbereich zwischen 9,2 µm und 9,6 µm zu erfassen, der die erste Wellenlänge von etwa 9,2 µm umfasst und ferner eine Wellenlänge von etwa 9,4 µm und etwa 9,6 µm umfasst,
und
wobei die Sensoreinheit mindestens einen zweiten Sensor (4) umfasst, der so ausgelegt ist, dass er IR-Strahlung mit mindestens einer zweiten Wellenlänge oder einem zweiten Wellenlängenbereich erfasst, wo die Intensität der erfassten IR-Strahlung im Wesentlichen unabhängig von der Glukosekonzentration in der Körperflüssigkeit des Subjekts ist.

2. System nach Anspruch 1, das keine externe Strahlungsquelle umfasst, die dazu ausgelegt ist, IR-Strahlung im Wellenlängenbereich von 5 µm bis 12 µm zu emittieren.

3. System nach Anspruch 1 oder 2, wobei die Körperflüssigkeit Blut ist.

4. System nach einem der vorhergehenden Ansprüche, wobei die Strahlungsquelle (a) so ausgelegt ist, dass sie VIS/NIR-Strahlung im Bereich von 550 nm bis 1200 nm, insbesondere im Bereich von 800 nm bis 820 nm, z.B. bei etwa 810 nm, und/oder im Bereich von 590 nm bis 610 nm, z.B. bei etwa 600 nm, und/oder im Bereich von 920 nm bis 980 nm, z.B. bei etwa 940 nm, emittiert.

5. System nach einem der vorhergehenden Ansprüche, wobei die Strahlungsquelle (a) eine LED, eine Laserdiode, ein VCSEL (Oberflächenemitter, vertical-cavity surface-emitting laser) oder ein Laser ist, und/oder wobei die Strahlungsquelle (a) eine Multiwellenlängen-Strahlungsquelle ist.

6. System nach einem der vorhergehenden Ansprüche, das ferner mindestens einen dritten Sensor umfasst,
wobei der mindestens eine dritte Sensor (i) dazu ausgelegt ist, unspezifische IR-Strahlung zu erfassen, (ii) dazu ausgelegt ist, unspezifische VIS/NIR-Strahlung zu erfassen, (iii) dazu ausgelegt ist, VIS/NIR-Strahlung mit einer Wellenlänge zu erfassen, bei der die Intensität der erfassten VIS/NIR-Strahlung von der Konzentration des physiologischen Parameters in der Körperflüssigkeit des Subjekts abhängig ist, und/oder (iv) ein Temperatursensor ist, der dazu ausgelegt ist, die Temperatur des Körperteils zu messen.

7. System nach einem der vorhergehenden Ansprüche, umfassend mindestens zwei verschiedene zweite Sensoren, die dazu ausgelegt sind, IR-Strahlung mit mindestens zwei verschiedenen Wellenlängen oder Wellenlängenbereichen zu erfassen,
wobei das System insbesondere zur Bestimmung von Glukose im Blut geeignet ist, wobei ein zweiter Sensor dazu ausgelegt ist, IR-Strahlung mit einer Wellenlänge oder einem Wellenlängenbereich zwischen 8,6 µm und 9,0 µm zu erfassen, und ein anderer zweiter Sensor dazu ausgelegt ist, IR-Strahlung mit einer Wellenlänge oder einem Wellenlängenbereich zwischen 9,8 µm und 10,2 µm zu erfassen.

8. System nach einem der vorhergehenden Ansprüche, wobei die Sensoreinheit (b) mindestens einen Sensor umfasst, der dazu ausgelegt ist, dass er zeitabhängig und separat IR-Strahlung mit unterschiedlichen Wellenlängen oder Wellenlängenbereichen erfasst,
wobei der Sensor dazu ausgelegt ist, in mindestens einem ersten Zeitintervall IR-Strahlung mit mindestens einer Wellenlänge oder einem Wellenlängenbereich zu erfassen, in dem die Intensität der erfassten IR-Strahlung von der Glukosekonzentration in der Körperflüssigkeit des Subjekts abhängig ist, und
wobei der Sensor dazu ausgelegt ist, in mindestens einem zweiten Zeitintervall IR-Strahlung mit mindestens einer Wellenlänge oder einem Wellenlängenbereich zu erfassen, in dem die Intensität der erfassten IR-Strahlung im Wesentlichen unabhängig von der Glukosekonzentration in der Körperflüssigkeit des Subjekts ist.

9. System nach einem der vorhergehenden Ansprüche,
wobei die Sensoreinheit (b) mindestens einen Sensor umfasst, der ein optischer Detektor, insbesondere ein optischer Photovoltaik-Detektor, noch spezieller ein Detektor auf InAsSb-Basis, ist.

10. System nach einem der vorhergehenden Ansprüche, das ferner eine Abdeckung umfasst, wobei die Abdeckung zumindest teilweise aus einem Material ausgebildet ist, das für die von der Strahlungsquelle (a) emittierte IR/VIS-Strahlung und für die von der Sensoreinheit (b) erfasste IR-Strahlung optisch transparent ist,
wobei die Abdeckung zumindest teilweise aus CaF₂ und/oder BaF₂ und/oder aus einem Kunststoffmaterial ausgebildet ist, das für IR-Strahlung transparent ist und optional für VIS/NIR-Strahlung transparent ist,
wobei das optisch transparente Material der Abdeckung eine Dicke von etwa 0,2 mm bis etwa 2 mm, insbesondere von etwa 0,5 mm bis etwa 1,5 mm, noch spezieller etwa 1 mm, aufweist, und/oder ferner eine Abdeckung umfasst, die IR-Strahlung von dem Körperteil auf die Sensoreinheit (b), insbesondere auf den mindestens einen Sensor der Sensoreinheit (b), fokussiert, insbesondere wobei die Abdeckung eine IR-Fresnellinse oder eine Anordnung aus mehreren IR-Fresnellinsen umfasst.

11. Verfahren zur nicht-invasiven Bestimmung von Glukose in einer Körperflüssigkeit eines Subjekts, umfassend die Schritte:
(a) Bestrahlen eines Körperteils (1) des Subjekts mit sichtbarer (VIS)/naher Infrarot (NIR)-Strahlung im Wellenlängenbereich von 400 nm bis 1500 nm oder 500 nm bis 1500 nm, wobei das bestrahlte Körperteil (1) die emittierte elektromagnetische Energie absorbiert, was zu einem lokalen Anstieg der Gewebetemperatur und zu einer erhöhten Emission von IR-Strahlung im Wellenlängenbereich von 5 µm bis 12 µm führt,
(b) Erfassen der von dem zuvor bestrahlten Körperteil des Subjekts emittierten IR-Strahlung im Wellenlängenbereich von 5 µm bis 12 µm,
umfassend das getrennte (i) Erfassen von IR-Strahlung mit mindestens zwei Wellenlängen oder Wellenlängenbereichen, wo die Intensität der erfassten IR-Strahlung von der Glukosekonzentration in der Körperflüssigkeit des Subjekts abhängt, umfassend das Erfassen von IR-Strahlung mit einer Wellenlänge von etwa 9,2 µm und das Erfassen von IR-Strahlung mit einem Wellenlängenbereich zwischen 9,2 µm und 9,6 µm, der die Wellenlänge von etwa 9,2 µm umfasst und ferner eine Wellenlänge von etwa 9,4 µm und etwa 9,6 µm umfasst,
und
(ii) Erfassen von IR-Strahlung mit mindestens einer Wellenlänge oder einem Wellenlängenbereich, wo die Intensität der erfassten IR-Strahlung im Wesentlichen unabhängig von der Glukosekonzentration in der Körperflüssigkeit des Subjekts ist, und
(c) Analysieren der erfassten IR-Strahlung zur qualitativen und/oder quantitativen Bestimmung von Glukose,
insbesondere wobei die Glukosekonzentration quantitativ bestimmt wird, und/oder wobei die Änderungsrate der Glukosemenge bestimmt wird, insbesondere nichtquantitativ bestimmt.

12. Verfahren nach Anspruch 11, wobei das Körperteil nicht mit einer externen Quelle von IR-Strahlung im Wellenlängenbereich von 5 µm bis 12 µm bestrahlt wird.

13. Verfahren nach Anspruch 11 oder 12, wobei die Körperflüssigkeit Blut ist.

## Revendications

1. Un système non invasif pour déterminer le glucose dans un fluide corporel d'un sujet comprenant :
(a) une source de rayonnement (9, 9a, 9b) adaptée pour émettre un rayonnement visible (VIS)/infrarouge proche (NIR) dans la plage de 400 nm à 1500 nm ou de 500 nm à 1500 nm dans une partie du corps (1) dudit sujet,
dans lequel la partie du corps (1) est particulièrement choisie parmi le bout d'un doigt, un lobe d'oreille, un poignet, un avant-bras et un bras,
et dans lequel la partie du corps irradiée absorbe l'énergie électromagnétique émise, ce qui entraîne une augmentation locale de la température du tissu et une augmentation de l'émission de rayonnement IR dans la plage de longueurs d'onde de 5 µm à 12 µm,
(b) une unité de détection adaptée pour détecter le rayonnement IR émis par la partie du corps précédemment irradiée dudit sujet dans la plage de 5 µm à 12 µm, dans lequel ladite unité de détection est adaptée pour (i) détecter le rayonnement IR ayant au moins une première longueur d'onde ou une première plage de longueurs d'onde où l'intensité du rayonnement détecté dépend de la concentration de glucose dans le fluide corporel dudit sujet, et pour (ii) détecter un rayonnement IR ayant au moins une deuxième longueur d'onde ou une deuxième plage de longueurs d'onde où l'intensité du rayonnement IR détecté est substantiellement indépendante de la concentration de glucose dans le fluide corporel dudit sujet,
dans lequel au moins ladite première longueur d'onde ou ladite première plage de longueurs d'onde et au moins ladite deuxième longueur d'onde ou ladite deuxième plage de longueurs d'onde se situent dans la plage de longueurs d'onde de 5 µm à 12 µm, et
(c) une unité d'analyse configurée pour effectuer la détermination qualitative et/ou quantitative du glucose sur la base du rayonnement IR détecté dans l'unité de détection (b) dans ladite moins une première longueur d'onde ou ladite première plage de longueurs d'onde et dans ladite au moins une deuxième longueur d'onde ou une deuxième plage de longueurs d'onde, dans lequel l'unité de détection (b) comprend au moins deux premiers capteurs (4),
dans lequel chacun des au moins deux capteurs (4) est adapté pour détecter un rayonnement IR ayant au moins une première longueur d'onde ou une première plage de longueurs d'onde où l'intensité du rayonnement IR détecté dépend de la concentration de glucose dans le fluide corporel dudit sujet, et
dans lequel au moins un premier capteur (4) est adapté pour détecter un rayonnement IR ayant une longueur d'onde d'environ 9,2 µm et au moins un autre premier capteur (4) est adapté pour détecter un rayonnement IR ayant une plage de longueurs d'onde comprise entre 9,2 µm et 9,6 µm qui comprend la première longueur d'onde d'environ 9,2 µm et comprend en outre une longueur d'onde d'environ 9,4 µm et d'environ 9,6 µm,
et
dans lequel l'unité de détection comprend au moins un deuxième capteur (4), qui est adapté pour détecter un rayonnement IR ayant au moins une deuxième longueur d'onde ou une deuxième plage de longueurs d'onde où l'intensité du rayonnement IR détecté est sensiblement indépendante de la concentration de glucose dans le fluide corporel dudit sujet.

2. Le système selon la revendication 1, qui ne comprend pas de source de rayonnement externe adaptée pour émettre un rayonnement IR dans la plage de longueurs d'onde de 5 µm à 12 µm.

3. Le système selon la revendication 1 ou 2, dans lequel le fluide corporel est du sang.

4. Le système selon l'une quelconque des revendications précédentes, dans lequel la source de rayonnement (a) est adaptée pour émettre un rayonnement VIS/NIR dans la plage de 550 nm à 1200 nm, en particulier dans la plage de 800 nm à 820 nm, par exemple d'environ 810 nm, et/ou dans la plage de 590 nm à 610 nm, par exemple d'environ 600 nm, et/ou dans la plage de 920 nm à 980 nm, par exemple d'environ 940 nm.

5. Le système selon l'une quelconque des revendications précédentes, dans lequel la source de rayonnement (a) est une LED, une diode laser, un vcsel (laser à cavité verticale émettant par la surface) ou un laser, et/ou dans lequel la source de rayonnement (a) est une source de rayonnement à longueurs d'onde multiples.

6. Le système selon l'une quelconque des revendications précédentes, comprenant en outre au moins un troisième capteur,
dans lequel ledit au moins un troisième capteur (i) est adapté pour détecter un rayonnement IR non spécifique (ii), est adapté pour détecter un rayonnement VIS/NIR non spécifique (iii), est adapté pour détecter un rayonnement VIS/NIR ayant une longueur d'onde, où l'intensité du rayonnement VIS/NIR détecté dépend de la concentration du paramètre physiologique dans le fluide corporel dudit sujet, et/ou (iv) est un capteur de température adapté pour mesurer la température de la partie du corps.

7. Le système selon l'une quelconque des revendications précédentes comprenant au moins deux deuxièmes capteurs différents, qui sont adaptés pour détecter un rayonnement IR ayant au moins deux longueurs d'onde ou plages de longueurs d'onde différentes,
dans lequel le système est particulièrement adapté pour déterminer le glucose dans le sang,
dans lequel un deuxième capteur est adapté pour détecter un rayonnement IR ayant une longueur d'onde ou une plage de longueurs d'onde comprise entre 8,6 µm et 9,0 µm et un autre deuxième capteur est adapté pour détecter un rayonnement IR ayant une longueur d'onde ou une plage de longueurs d'onde comprise entre 9,8 µm et 10,2 µm.

8. Le système selon l'une des revendications précédentes, dans lequel l'unité de détection (b) comprend au moins un capteur adapté pour détecter séparément et en fonction du temps un rayonnement IR ayant différentes longueurs d'onde ou plages de longueurs d'onde,
dans lequel, dans au moins un premier intervalle de temps, le capteur est adapté pour détecter un rayonnement IR ayant au moins une longueur d'onde ou une plage de longueurs d'onde où l'intensité du rayonnement IR détecté dépend de la concentration de glucose dans le fluide corporel dudit sujet, et
dans lequel, dans au moins un deuxième intervalle de temps, le capteur est adapté pour détecter un rayonnement IR ayant au moins une longueur d'onde ou une plage de longueurs d'onde où l'intensité du rayonnement IR détecté est substantiellement indépendante de la concentration de glucose dans le fluide corporel dudit sujet.

9. Le système selon l'une quelconque des revendications précédentes,
dans lequel l'unité de détection (b) comprend au moins un capteur qui est un détecteur optique, en particulier un détecteur optique photovoltaïque, plus particulièrement un détecteur à base d'InAsSb.

10. Le système selon l'une quelconque des revendications précédentes comprenant en outre un couvercle, dans lequel ledit couvercle est au moins partiellement constitué d'un matériau optiquement transparent pour le rayonnement IR/VIS émis par la source de rayonnement (a) et pour le rayonnement IR détecté par l'unité de détection (b),
dans lequel le couvercle est au moins partiellement constitué de CaF₂ et/ou de BaF₂ et/ou d'un matériau plastique qui est transparent pour le rayonnement IR et éventuellement transparent pour le rayonnement VIS/NIR,
dans lequel le matériau optiquement transparent du couvercle a une épaisseur d'environ 0,2 mm à environ 2 mm, en particulier d'environ 0,5 mm à environ 1,5 mm, plus particulièrement d'environ 1 mm, et/ou comprenant en outre un couvercle qui concentre le rayonnement IR de la partie du corps vers l'unité de détection (b), en particulier vers ledit au moins un capteur de l'unité de détection (b), en particulier dans lequel le couvercle comprend une lentille de Fresnel IR ou un réseau comprenant une pluralité de lentilles de Fresnel IR.

11. Une méthode de détermination non invasive du glucose dans un fluide corporel d'un sujet, comprenant les étapes suivantes :
(a) irradier une partie du corps (1) dudit sujet avec un rayonnement visible (VIS)/infrarouge proche (NIR) dans la plage de longueurs d'onde de 400 nm à 1500 nm, ou de 500 nm à 1500 nm, dans lequel la partie du corps irradiée (1) absorbe l'énergie électromagnétique émise, ce qui entraîne une augmentation locale de la température des tissus et une émission augmentée de rayonnement IR dans la plage de longueurs d'onde de 5 µm à 12 µm,
(b) détecter le rayonnement IR émis par la partie du corps dudit sujet préalablement irradiée dans la plage de longueurs d'onde de 5 µm à 12 µm,
comprenant séparément (i) la détection du rayonnement IR ayant au moins deux longueurs d'onde ou plages de longueurs d'onde où l'intensité du rayonnement IR détecté dépend de la concentration de glucose dans le fluide corporel dudit sujet, comprenant la détection du rayonnement IR ayant une longueur d'onde d'environ 9,2 µm et la détection d'un rayonnement IR ayant une plage de longueurs d'onde comprise entre 9,2 µm et 9,6 µm qui comprend la longueur d'onde d'environ 9,2 µm et comprend en outre une longueur d'onde d'environ 9,4 µm et d'environ 9,6 µm, et
(ii) détecter le rayonnement IR ayant au moins une longueur d'onde ou une plage de longueurs d'onde où l'intensité du rayonnement IR détecté est substantiellement indépendante de la concentration de glucose dans le fluide corporel dudit sujet, et (c) analyser le rayonnement IR détecté pour la détermination qualitative et/ou quantitative du glucose,
en particulier dans lequel la concentration de glucose est déterminée quantitativement, et/ou dans laquelle le taux d'altération de la quantité de glucose est déterminé, en particulier de manière non quantitative.

12. La méthode selon la revendication 11, dans laquelle la partie du corps n'est pas irradiée par une source externe de rayonnement IR dans la plage de longueurs d'onde de 5 µm à 12 µm.

13. La méthode selon la revendication 11 ou 12, dans laquelle le fluide corporel est du sang.
